# EUROPÄISCHE PATENTSCHRIFT

(11) **EP 3 914 172 B1**
(45) Veröffentlichungstag und Bekanntmachung des Hinweises auf die Patenterteilung: **08.03.2023**
(21) Anmeldenummer: 20701580.1
(22) Anmeldetag: 21.01.2020
(51) Int. Cl.: A61B 17/72

(54) **GEKRÜMMTER MARKNAGEL**
CURVED INTRAMEDULLARY NAIL
CLOU MÉDULLAIRE INCURVÉ

(30) Priorität: 24.01.2019 DE 102019000538
(43) Veröffentlichungstag der Anmeldung: 01.12.2021
(73) Patentinhaber: Betz, Augustin, 66687 Wadern-Wadrill (DE)
(72) Erfinder: Betz, Augustin, 66687 Wadern-Wadrill (DE)
(74) Vertreter: Manitz Finsterwald Patent- und Rechtsanwaltspartnerschaft mbB
(86) Internationale Anmeldenummer: PCT/EP2020/051420
(87) Internationale Veröffentlichungsnummer: WO 2020/152171

(56) Entgegenhaltungen:
- WO-A1-98/47438
- US-A- 5 074 882

## Beschreibung

Die Erfindung betrifft einen Marknagel, insbesondere einen Distraktionsmarknagel oder Kontraktionsmarknagel, zum Verbinden zweier Teile eines Knochens, insbesondere zur Knochenverlängerung, zur Knochenverkürzung oder zur Überbrückung einer Knochenlücke, welcher ein Rohr zum Befestigen an dem ersten Knochenteil, eine innerhalb des Rohres angeordneten Stange, welche einen aus dem Rohr herausragenden Befestigungsabschnitt zum Befestigen an dem zweiten Knochenteil aufweist, und eine innerhalb des Rohres angeordnete Einstellmechanik umfasst, welche durch wiederholte relative Rotationsbewegungen zwischen der Stange und dem Rohr betätigbar ist, wobei die Stange durch Betätigen der Einstellmechanik aus dem Rohr herausfahrbar oder in das Rohr hineinfahrbar ist.

Insbesondere Distraktionsmarknägel sind Stand der medizinischen Technik und bekannt unter anderem aus den Druckschriften DE19700225A1, DE3921972A1, US9179938B2 und CA2917676A1. Diese Vorrichtungen dienen zur Verlängerung von Röhrenknochen oder zur Defektüberbrückung nach Trümmerbrüchen, einer Knochenentzündung oder nach Entfernen von Tumoren im Bereich langer Röhrenknochen. Distraktionsmarknägel weisen erhebliche Vorteile gegenüber äußeren Distraktionsvorrichtungen auf, bei denen die Knochenteile durch die Haut nach außen mit einem verstellbaren Rahmen verbunden sind, wodurch zum einen ein ungünstiger Kraftansatz vorliegt und zum anderen eine ständige Infektionsgefahr durch das Eindringen von Keimen besteht.

Die Funktionsweise bekannter Distraktionsmarknägel ist derart, dass die beiden Teile des durchtrennten Knochens schrittweise durch die Verlängerung des an ihnen befestigten Marknagels auseinander bewegt werden und die dadurch entstehende Lücke zwischen den beiden Knochenenden fortlaufend durch neu gebildete Knochensubstanz überbrückt wird. Dabei können die Knochenteile axial mittels einer vom Arzt oder Patienten bedienbaren, sich im Inneren des Marknagels befindlichen Einstellmechanik auseinander bewegt werden. Marknägel können ferner auch als Kontraktionsmarknägel ausgebildet sein, bei welchen eine Betätigung der Einstellmechanik eine Verkürzung des Abstandes der mit dem Marknagel verbundenen Knochenteile zur Folge hat.

Als besonders vorteilhaft bzgl. Robustheit, Zuverlässigkeit und Korrosionsbeständigkeit haben sich Distraktionsmarknägel entsprechend der Druckschrift US5074882 erwiesen. Hierbei erfolgt die Verlängerung des Marknagels durch ein wiederholtes Verdrehen und Zurückdrehen des Marknagels um einen definierten Winkel, ähnlich der Funktionsweise einer Ratsche. Grundsätzlich kann auch ein Kontraktionsmarknagel auf solche Art ausgebildet werden, indem beispielsweise die Drehrichtung der Rotationsbewegungen bei einem Distraktionsmarknagel wie in US5074882 offenbart umgekehrt wird. Bis dato sind jedoch ausschließlich Marknägel verfügbar, die über die Gesamtlänge geradlinig ausgeführt sind und die eine geradlinige Verlängerung durchführen.

Solche geradlinigen Marknägel können sich jedoch häufig aufgrund physiologischer Krümmungen zu verlängernder Röhrenknochen als operativ aufwendig einzusetzen erweisen und es kann, in der Regel, im Zuge einer Operation zu einer Schwächung der Kortikalis kommen.

Es ist daher eine Aufgabe der Erfindung, einen Marknagel zu schaffen, welcher eine Anpassung an eine physiologische Krümmung eines Knochens ermöglicht und dabei eine hohe Belastbarkeit aufweist.

Diese Aufgabe wird gelöst durch einen Marknagel mit den Merkmalen des Anspruchs 1 und insbesondere dadurch, dass der Marknagel als gekrümmter Marknagel, insbesondere mit einer der physiologischen Krümmung des Knochens entsprechenden Krümmung, ausgebildet ist, und ferner insbesondere dadurch, dass eine Entkopplung zwischen der Stange und zumindest einer Komponente der Einstellmechanik vorgesehen ist, welche die Übertragung von krümmungsbedingten Biegemomenten auf die Komponente verhindert.

Insbesondere kann die Einstellmechanik dabei durch wiederholtes Vor- und Zurückdrehen, ähnlich wie bei einer Ratsche, betätigt werden, um die Länge des Befestigungsabschnitts der Stange, welcher aus dem Rohr herausragt, inkrementell zu verändern. Dadurch kann entsprechend die Länge des Marknagels verändert und je nach Ausbildung als Distraktionsmarknagel oder Kontraktionsmarknagel verlängert oder verkürzt werden, sodass auch die beiden durch den Marknagel verbundenen Knochenteile auseinander oder aufeinander zu bewegt werden können.

Um mittels der Einstellmechanik die Stange aus dem Rohr heraus- oder in das Rohr hineinfahren zu können, ist grundsätzlich eine Kopplung zwischen der Stange und der Einstellmechanik erforderlich. Bei einer gekrümmten Ausbildung der Stange bzw. des Rohres besteht dabei die Problematik, dass aufgrund dieser Krümmung Biegemomente auf die Einstellmechanik bzw. deren Komponenten übertragen werden können. Diese Biegemomente können insbesondere bei der Betätigung der Einstellmechanik oder einer Belastung des Marknagels, beispielsweise während eines Stehens eines Patienten mit einem mittels eines Distraktionsmarknagels verlängerten Beinknochen, zu einer Beschädigung der Einstellmechanik oder sogar einem Brechen der jeweiligen Komponenten führen. Insbesondere weisen bekannte Einstellmechaniken häufig Schraubverbindungen auf, wobei die Schrauben als geradlinige Komponenten besonders der beschriebenen Gefahr eines krümmungsbedingten Brechens ausgesetzt sein können. Eine solche Beschädigung oder ein Brechen kann jedoch die Funktionalität der Einstellmechanik derart beeinträchtigen oder sogar vollständig ausschließen, sodass die Behandlung nicht fortgeführt oder eine weitere Operation zum Einsetzen eines neuen Marknagels erforderlich sein kann. Insbesondere diese Gefahr hat den Einsatz gekrümmter Marknägel bisher stets unmöglich gemacht.

Durch die Entkopplung zwischen der Stange und zumindest einer Komponente der Einstellmechanik bezüglich der Übertragung von krümmungsbedingten Biegemomenten kann dieser Gefahr begegnet und ein zuverlässig hoch belastbarer Marknagel mit einer Krümmung ausgebildet werden, welche insbesondere der physiologischen Krümmung des zu behandelnden Knochens entsprechen kann. Das Einbringen des Marknagels in den Knochen kann dadurch für den Chirurgen erheblich erleichtert werden und der Marknagel kann den Markraum des Knochens optimal ausnutzen. Ferner kann dadurch die Kortikalis geschont werden. Krümmungsbedingte, insbesondere während des Verlängerungs- oder Verkürzungsprozesses des Knochens auftretende Belastungen werden dabei nicht auf die Einstellmechanik bzw. zumindest nicht auf die entkoppelte Komponente geleitet, so dass eine ausreichende und zuverlässige Belastbarkeit des gekrümmten Marknagels erreicht werden kann. Insbesondere kann die entkoppelte Komponente eine notwendige Schraubverbindung der Einstellmechanik sein.

Mögliche Ausführungsformen der Erfindung sind den abhängigen Ansprüchen, der Beschreibung und den Figuren zu entnehmen.

Bei einigen Ausführungsformen kann das Rohr ein Innengewinde aufweisen und die Einstellmechanik kann ein in dem Innengewinde um eine Drehachse drehbares Gewindeelement umfassen. Dabei kann, in Abhängigkeit von der Gewindesteigung des Innengewindes bzw. der Drehrichtung des Gewindeelements, durch ein Drehen des Gewindeelements eine axiale Bewegung des Gewindeelements in Richtung der Stange oder von der Stange weg erreicht werden, sodass die Stange durch ein Zusammenwirken des Gewindeelements mit der Stange aus dem Rohr herausgeschoben oder ein Hineinfahren der Stange in das Rohr ermöglicht werden kann. Dabei kann die Stange mit dem Gewindeelement verbunden sein, so dass die Stange bei einem Kontraktionsmarknagel durch das Gewindeelement in das Rohr hineinbewegt werden kann. Aufgrund der Krümmung des Marknagels kann sich die Ausrichtung der Drehachse dabei während eines Herausfahrens oder Hineinfahrens der Stange abhängig von der Position des Gewindeelements verändern.

Das Gewindeelement kann über eine erste Verzahnung mit der Stange in Eingriff stehen. Die Verzahnung kann dabei direkt von einem Ende der Stange gebildet sein oder die Stange kann mittelbar über ein weiteres Element mit dem Gewindeelement in Eingriff stehen, wobei das weitere Element die Verzahnung bilden kann. Insbesondere kann durch einen solchen Eingriff eine Rotationsbewegung der Stange auf das Gewindeelement übertragen werden, um eine axiale bzw. translatorische Verschiebung des Gewindeelements und der mit diesem in Eingriff stehenden Stange erreichen zu können.

Eine Rotationsbewegung der Stange um die Drehachse entlang einer Drehrichtung kann über die erste Verzahnung auf das Gewindeelement übertragbar sein. Beispielsweise kann eine Rotationsbewegung eines mit der Stange verbundenen Knochenteils über die Stange auf das Gewindeelement übertragen werden, um ein Aus- oder Einfahren des Gewindeelements und der Stange aus dem bzw. in das Rohr, je nach Gewindesteigung bzw. Drehrichtung des Gewindeelementes, zu erreichen.

Bei einigen Ausführungsformen kann die Stange eine Passfeder aufweisen, welche in einer Nut des Rohres geführt ist, oder umgekehrt, wobei die Nut die Rotationsbewegung der Stange um die Drehachse begrenzen kann. Insbesondere kann die Nut dabei eine Rotationsbewegung der Stange in Drehrichtung begrenzen, so dass eine Bewegung der Stange zum Betätigen der Einstellmechanik begrenzt ist und ein Überdrehen über eine vorgegebene Drehstellung hinaus vermieden werden kann. Insbesondere kann es vorgesehen sein, dass der Ausgangszustand der Einstellmechanik durch ein Zurückdrehen der Stange erreichbar ist, wobei auch diese Rotationsbewegung der Stange entgegen der Drehrichtung durch die Nut und die Passfeder entsprechend begrenzt werden kann. Dadurch kann der Umfang der Rotationsbewegungen zur Betätigung der Einstellmechanik klar definiert werden, um bei jeder Betätigung der Einstellmechanik eine vorgegebene und überprüfbare bzw. dokumentierbare Veränderung der Länge des Marknagels erhalten zu können.

Bei einigen Ausführungsformen kann das Gewindeelement an einer der Stange abgewandten Seite über eine zweite Verzahnung mit einem Blockierelement in Eingriff stehen, wobei die zweite Verzahnung entgegen der Drehrichtung eine Sperrrichtung aufweisen kann, welche eine Rotation des Gewindeelements relativ zu dem Blockierelement entgegen der Drehrichtung blockiert, und wobei die zweite Verzahnung in Drehrichtung eine Freilaufrichtung aufweisen kann, welche eine Rotation des Gewindeelements relativ zu dem Blockierelement in Drehrichtung ermöglicht. Während eines von der Stange durch den Eingriff der ersten Verzahnung übermittelten Drehens des Gewindeelements in Drehrichtung kann die zweite Verzahnung somit freilaufend einen Zahn überspringen, um die Drehung des Gewindeelements und die damit einhergehende translatorische Bewegung desselben und der mit dem Gewindeelement in Eingriff stehenden Stange zu ermöglichen. Beim Zurückdrehen der Stange entgegen der Drehrichtung kann das Blockierelement das Gewindeelement hingegen an einem Mitdrehen hindern, sodass die erfolgte translatorische Verschiebung bestehen bleibt. Die Einstellmechanik kann somit nach dem Prinzip einer Ratsche funktionieren.

Die erste Verzahnung kann entgegen der Drehrichtung eine Freilaufrichtung aufweisen, welche ein Zurückdrehen der Stange entgegen der Drehrichtung bei durch das Blockierelement blockiertem Gewindeelement ermöglicht. Dabei kann die erste Verzahnung beim Zurückdrehen der Stange freilaufend einen Zahn überspringen, so dass eine Rotation der Stange lediglich in Drehrichtung, nicht jedoch entgegen dieser auf das Gewindeelement übertragen werden kann. Während einer Drehung der Stange in Drehrichtung kann diese folglich auf das Gewindeelement übertragen werden, wobei sich das Gewindeelement aufgrund der Gewindesteigung des Innengewindes auch translatorisch bewegt. Bei einem darauffolgenden Zurückdrehen der Stange, um die Stange und den damit verbundenen Knochen wieder in die Ausgangslage zu überführen, kann das Gewindeelement hingegen in Ruhe verbleiben und entsprechend keine weitere axiale Positionsveränderung erfahren. Daraufhin kann die Einstellmechanik erneut durch Vor- und Zurückdrehen der Stange betätigt werden.

Das Blockierelement kann verdrehsicher bezüglich der Drehachse innerhalb des Rohres geführt sein, wobei das Blockierelement insbesondere eine Passfeder aufweisen kann, welche innerhalb des Rohres in einer Nut geführt ist, oder umgekehrt. Insbesondere kann es sich bei dieser Nut um die bereits erwähnte Nut handeln, die auch die Stange führt bzw. deren Rotationsbewegung begrenzt. Entsprechend kann die Passfeder der Stange in Umfangsrichtung weniger stark ausgedehnt sein als die Passfeder des Blockierelements, so dass die Stange um ein durch das Spiel zwischen der Passfeder und der Nut vorgegebenes Maß verdrehbar sein kann, während das Blockierelement in der Nut gegenüber Rotationsbewegungen fixiert sein kann. Insbesondere kann dadurch ein zuverlässiges Blockieren des Gewindeelements beim Zurückdrehen der Stange erreicht werden, so dass ein Verändern der durch das Drehen des Gewindeelements erfolgen Längenanpassung des Marknagels beim Zurückdrehen der Stange verhindert werden kann. Alternativ kann auch eine jeweilige gesonderte Nut zur Führung der Stange und des Blockierelements vorgesehen sein.

Das Blockierelement kann in Eingriff mit dem Gewindeelement vorgespannt und gegen die Rückstellkraft der Vorspannung außer Eingriff mit dem Gewindeelement bringbar sein. Hierdurch kann zunächst sichergestellt werden, dass das Blockierelement dem Gewindeelement bei einer translatorischen Bewegung folgt und in Eingriff verbleibt, um darauffolgend eine Drehung des Gewindeelements entgegen der Drehrichtung zu verhindern. Andererseits kann das Blockierelement während eines Drehens des Gewindeelements in Drehrichtung entgegen der Rückstellkraft der Vorspannung bewegbar sein, um den Freilauf und die Drehung des Gewindeelements zu ermöglichen.

Bei einigen Ausführungsformen kann das Blockierelement mittels eines geradlinigen Verbindungselements, welches sich durch das Blockierelement hindurch erstreckt, in Eingriff mit dem Gewindeelement vorgespannt sein. Das Verbindungselement, welches insbesondere als Schraube ausgebildet sein kann, kann sich dabei insbesondere durch das Gewindeelement hindurch oder in das Gewindeelements hinein erstrecken, wobei das Verbindungselement ferner in der Stange oder dem Gewindeelement verankert sein kann. Insbesondere die Übertragung von krümmungsbedingten Biegemomenten auf ein solches geradliniges Verbindungselement kann durch die Entkopplung verhindert werden, um ein eventuelles Brechen dieses Verbindungselementes zuverlässig verhindern zu können.

Zwischen dem Verbindungselement und dem Blockierelement kann eine Spanneinrichtung, insbesondere eine Feder, wirksam sein, welche die Vorspannung auf das Blockierelement ausübt. Durch die Vorspannung kann der Eingriff der zweiten Verzahnung sichergestellt werden, um ein Blockieren des Gewindeelements entgegen der Drehrichtung zu erreichen. Ferner kann durch die Verbindung des Gewindeelements und des Blockierelements erreicht werden, dass das Blockierelement etwaigen translatorischen Bewegungen des Gewindeelements folgt. Um die Vorspannung auf das Blockierelement übertragen zu können, kann das Verbindungselement beispielsweise in dem Gewindeelement und/oder einem weiteren Element verankert sein.

Das Verbindungselement kann sich parallel zu der Drehachse erstrecken, wobei eine Längsachse des Verbindungselements insbesondere mit der Drehachse zusammenfallen kann. Auch die Ausrichtung des Verbindungselements kann entsprechend von der Position des Gewindeelements und der krümmungsbedingten veränderlichen Ausrichtung der Drehachse zumindest geringfügig abhängen.

Bei einigen Ausführungsformen kann das Verbindungselement als Komponente der Einstellmechanik in dem Gewindeelement verankert sein und hierdurch die Entkopplung zwischen Stange und Verbindungselement erfolgen. Von der Stange beispielsweise infolge einer Rotations- oder Translationsbewegung während eines Betätigens der Einstellmechanik oder infolge einer Druckbelastung auf den Marknagel übertragene Biegemomente können somit über das Gewindeelement auf das Rohr abgeleitet werden. Indem das Verbindungselement, welches insbesondere als Schraube ausgebildet sein kann, jedoch nicht in der Stange selbst verankert ist, kann eine Belastung dieser Komponente durch solche Biegemomente verhindert werden.

Das Gewindeelement kann ferner über ein insbesondere stiftförmiges Zentrierelement mit der Stange verbunden sein, welches die Stange axial bezüglich der Drehachse zu dem Gewindeelement ausrichtet. Dadurch kann erreicht werden, dass die Stange und das Gewindeelement unabhängig von der Krümmung korrekt zueinander ausgerichtet verbleiben und die erste Verzahnung stets zuverlässig in Eingriff steht. Durch diesen Eingriff kann eine Übertragung einer Drehung der Stange auf das Gewindeelement unabhängig von der Position des Gewindeelements und einer gegebenenfalls positionsabhängigen Krümmung des Marknagels sichergestellt werden.

Bei einigen Ausführungsformen kann zur Entkopplung der Stange von dem eine Komponente der Einstellmechanik bildenden Verbindungselement ein Gelenk vorgesehen sein, welches zumindest einen rotatorischen Freiheitsgrad zwischen dem Verbindungselement und der Stange schafft. Auch durch einen solchen rotatorischen Freiheitsgrad kann die Krümmung der Stange bzw. des Rohres berücksichtigt und eine Abweichung der Führung der Stange während einer Längenanpassung des Marknagels gegenüber dem geradlinigen Verbindungselement ermöglicht werden, ohne dass dadurch Biegemomente auf das Verbindungselement, insbesondere eine Schraube, übertragen werden. Dabei können die Stange und das Verbindungselement unmittelbar gelenkig miteinander verbunden sein, während es auch möglich ist, dass die Stange gelenkig mit einem weiteren Element verbunden ist, in welchem das Verbindungselement beispielsweise verankert ist.

Das Gelenk kann zumindest einen rotatorischen Freiheitsgrad bezüglich Drehbewegungen um zumindest eine senkrecht zu der Drehachse verlaufende Achse schaffen. Insbesondere kann die Stange durch das Gelenk gegenüber dem Verbindungselement um eine senkrecht zu der Drehachse verlaufende Schwenkachse verschwenkbar sein. Eine solche Schwenkbewegung kann dabei durch das Rohr, innerhalb dessen die Stange angeordnet ist, beschränkt werden.

Das Gelenk kann die Stange mit einem Antriebselement drehfest bezüglich Rotationsbewegungen um die Drehachse verbinden.

Dabei kann das Antriebselement die erste Verzahnung mit dem Getriebeelement bilden. Bei solchen Ausführungsformen kann eine Drehung bzw. Rotationsbewegung der Stange folglich zunächst auf das Antriebselement übertragen werden, welches die Rotation über die erste Verzahnung auf das Getriebeelement überträgt. Während das Gelenk dabei eine insbesondere krümmungsbedingte Auslenkung der Stange gegenüber dem Antriebselement ermöglicht, kann das Antriebselement stets korrekt bzw. gegenüber dem Gewindeelement nicht ausgelenkt ausgerichtet sein, sodass die erste Verzahnung zwischen dem Antriebselement und dem Gewindeelement stets in Eingriff stehen und eine zuverlässige Übertragung einer Rotationsbewegung der Stange auf das Gewindeelement sichergestellt werden kann. Das Antriebselement sowie die weiteren Komponenten der Einstellmechanik können durch diese gelenkige Verbindung ferner von krümmungsbedingten Auslenkungen der Stange entkoppelt werden, so dass keinerlei Biegemomente auf die Komponenten der Einstellmechanik übertragen werden.

Das Antriebselement kann eine Passfeder aufweisen, welche in einer Nut des Rohres geführt ist, oder umgekehrt, wobei die Nut die Rotationsbewegung des Antriebselements in die Drehachse begrenzen kann. Insbesondere kann die Nut der bereits genannten Nut zur Begrenzung der Rotationsbewegung der Stange entsprechen oder es kann eine gesonderte Nut zur Führung des Antriebselementes vorgesehen sein. Das Antriebselement kann dabei alternativ oder zusätzlich zu der Stange eine, gegebenenfalls weitere, Passfeder aufweisen, um Rotationsbewegungen des Antriebselements und aufgrund der drehfesten Verbindung auch der Stange zu begrenzen.

Bei einigen Ausführungsformen kann sich das Verbindungselement durch das Blockierelement hindurch erstrecken und in dem Antriebselement verankert sein. Somit kann die gesamte Einstellmechanik durch das Verbindungselement miteinander verbunden sein, wobei durch die gelenkige Verbindung zwischen der Stange und dem Antriebselement eine Übertragung von Biegungsmomenten auf die Einstellmechanik und insbesondere das Verbindungselement verhindert werden kann.

Das Gelenk kann einen an der Stange ausgebildeten ersten Gelenkabschnitt und einen an dem Antriebselement ausgebildeten zweiten Gelenkabschnitt umfassen, wobei der erste Gelenkabschnitt formschlüssig mit dem zweiten Gelenkabschnitt verbunden sein kann. Dadurch kann die von dem Marknagel zu tragende Axialkraft, insbesondere während eines Stehens eines Patienten mit durch den Marknagel verbundenen Beinknochen, über die formschlüssige Anlage der beiden Gelenkabschnitte aneinander übertragen werden, so dass größtmögliche Flächen zu dieser Kraftübertragung genutzt werden und eine hohe Belastbarkeit des Marknagels erreicht werden kann.

Dabei kann der erste Gelenkabschnitt konvex und der zweite Gelenkabschnitt konkav, oder umgekehrt, ausgebildet sein, wobei der erste Gelenkabschnitt und der zweite Gelenkabschnitt im Querschnitt insbesondere kreisförmig ausgebildet sein können. Beispielsweise können die Gelenkflächen zylinderförmig bzw. als Teile, beispielsweise Hälften, einer Mantelfläche eines jeweiligen Zylinders geformt sein, wobei der konvex geformte Gelenkabschnitt von dem konkav geformten Gelenkabschnitt umgriffen sein kann. Dadurch kann zunächst ein rotatorischer Freiheitsgrad in Umfangsrichtung der Mantelflächen bzw. um eine senkrecht zu der Erstreckungsrichtung der Stange orientierte Zylinderachse geschaffen werden, um eine Auslenkung der Stange relativ zu dem Antriebselement um diese Achse zu ermöglichen. Bewegungen bzw. Auslenkungen der Stange können dabei durch eine Innenwand bzw. das Innengewinde des Rohres beschränkt sein. Eine solche Ausbildung der Gelenkabschnitte kann es ferner ermöglichen, eine Drehung der Stange um eine Achse senkrecht zu der genannten Zylinderachse zum Betätigen der Einstellmechanik unmittelbar über die aneinander anliegenden Gelenkabschnitte auf das Antriebselement zu übertragen.

Bei einigen Ausführungsformen kann die Stange über ein Kopplungselement, insbesondere einen Bolzen, mit dem Antriebselement verbunden sein. Insbesondere kann die Stange durch diesen Bolzen mit den Komponenten der Einstellmechanik verbunden sein, so dass beispielsweise ein Herausrutschen der Stange aus dem Rohr während der Montage bzw. dem Einsetzen des Marknagels verhindert werden kann. Grundsätzlich hat es sich jedoch herausgestellt, dass es auch möglich ist, ein mit einem Gelenk ausgebildeten Marknagel ohne ein solches Kopplungselement vorzusehen, wobei der zuverlässige Zusammenhalt des eingesetzten Marknagels über die Muskelspannung erreicht werden kann.

Das Kopplungselement kann mit Spiel in der Stange und/oder dem Antriebselement gelagert sein. Dadurch kann insbesondere erreicht werden, dass keine axialen bzw. in einer Längsrichtung des Marknagels wirkenden Kräfte über das Kopplungselement übertragen werden, durch welche das Kopplungselement belastet und gegebenenfalls beschädigt werden könnte. Die Übertragung solcher axialen Kräfte kann vielmehr beispielsweise durch ein formschlüssiges Anliegen eines von der Stange gebildeten Gelenkabschnitts an einem von dem Antriebselement gebildeten Gelenkabschnitt erfolgen.

Das Kopplungselement kann zur drehfesten Kopplung der Stange mit dem Antriebselement vorgesehen sein. Beispielsweise kann das Antriebselement die Stange gabelartig übergreifen, wobei ein als Bolzen ausgebildetes Kopplungselement durch die Gabelabschnitte des Antriebselementes und die Stange hindurch geführt sein kann, um die Stange und das Antriebselement drehfest zu verbinden.

Bei einigen Ausführungsformen kann das Gelenk einen polygonalen Kopf umfassen, welcher mit der Stange verbunden und in einer an dem Antriebselement ausgebildeten Ausnehmung gegen die Drehachse verschwenkbar gelagert sein kann. Beispielsweise kann dazu eine in die Stange eingeschraubte Sechskantschraube vorgesehen sein, welche mit Spiel in einem an dem Antriebselement ausgebildeten Innensechskant gelagert ist. Dabei kann ein solches Spiel derart gewählt sein, dass eine Rotationsbewegung der Stange um die Drehachse über den polygonalen Kopf und die Ausnehmung auf das Antriebselement übertragbar ist, während eine krümmungsbedingte Auslenkung der Stange gegenüber dem Antriebselement und eine entsprechende Auslenkung des polygonalen Kopfes in der Ausnehmung ermöglicht werden. Durch diese Auslenkung des polygonalen Kopfes in der Ausnehmung kann folglich eine Entkopplung der Stange von der Einstellmechanik bzw. dem Antriebselement hinsichtlich der Übertragung von Biegemomenten erfolgen.

Ferner kann das Gelenk als Kardangelenk ausgebildet sein.

Bei einigen Ausführungsformen kann die Stange mit einem radialen Spiel innerhalb des Rohres angeordnet sein. Dadurch kann die Stange auch bei einer nicht über die gesamte Länge des Rohres gleichbleibenden Krümmung in das Rohr hinein- oder aus diesem herausgefahren werden, um die Länge des gekrümmten Marknagels anzupassen.

Die Rotations- oder Translationsbewegungen zum Betätigen der Einstellmechanik können manuell erzeugbar sein, insbesondere dann, wenn das Rohr und die Stange an den jeweiligen Knochenteilen befestigt sind. Zum Betätigen der Einstellmechanik und entsprechend zur Anpassung der Länge des Marknagels sind folglich keine Antriebe oder Motoren, die sich als fehler- und störungsanfällig erweisen können, erforderlich. Vielmehr kann es beispielsweise vorgesehen sein, die Rotationsbewegung zur Verlängerung eines Unterschenkels durch ein manuelles Drehen an dem unteren Knochenteil bzw. dem Fuß eines Patienten auf die Einstellmechanik bzw. das Gewindeelement zu übertragen. Insbesondere können diese Handlungen auch unkompliziert von dem Patienten selbst vorgenommen werden.

Die Erfindung wird im Folgenden rein beispielhaft anhand von Ausführungsformen unter Bezugnahme auf die Zeichnungen erläutert.

Es zeigen:
- Fig. 1A: eine schematische Darstellung eines gekrümmten Marknagels,
- Fig. 1B: eine schematische Detailansicht des Marknagels zur Veranschaulichung der Entkopplung zwischen der Stange und einer Komponente der Einstellmechanik, und
- Fig. 2 und 3: jeweilige schematische Detailansichten weiterer Ausführungsformen gekrümmter Marknagel zur Veranschaulichung der Entkopplung zwischen der Stange und einer Komponente der Einstellmechanik.

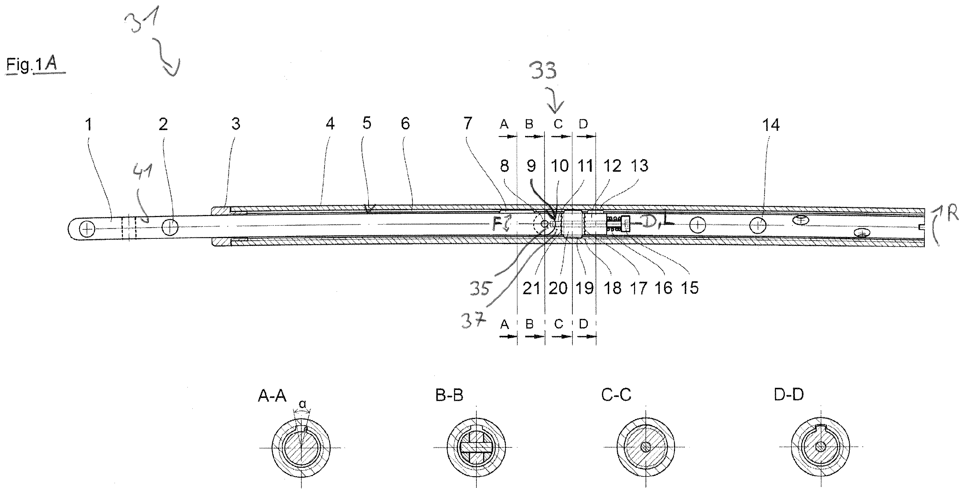

Fig. 1A zeigt einen gekrümmten Marknagel 31 und insbesondere einen Distraktionsmarknagel entsprechend Druckschrift US5074882, welcher um die Details eines radialen Spiels 5, eines Kopplungselements 8, eines Gelenks 9 und eines Antriebselements 10 weitergebildet ist. Diese Details ermöglichen eine gekrümmte Ausführung des Marknagels, wie nachfolgend weiter beschrieben ist.

Der gekrümmte Marknagel 31 umfasst eine Stange 1, welche innerhalb eines Rohres 4 angeordnet ist. Dabei ragt ein Befestigungsabschnitt 41 der Stange 1 aus dem Rohr 4 heraus, welcher mit Bohrungen 2 versehen ist, durch welche die Stange 1 mittels nicht gezeigter Schrauben an einem ersten Teil eines Röhrenknochens befestigt werden kann. Auch das Rohr 4 weist an der dem Befestigungsabschnitt 41 der Stange 1 gegenüberliegenden Abschnitt Bohrungen 14 zur Befestigung des Rohres 4 an einem zweiten Teil eines Röhrenknochens auf.

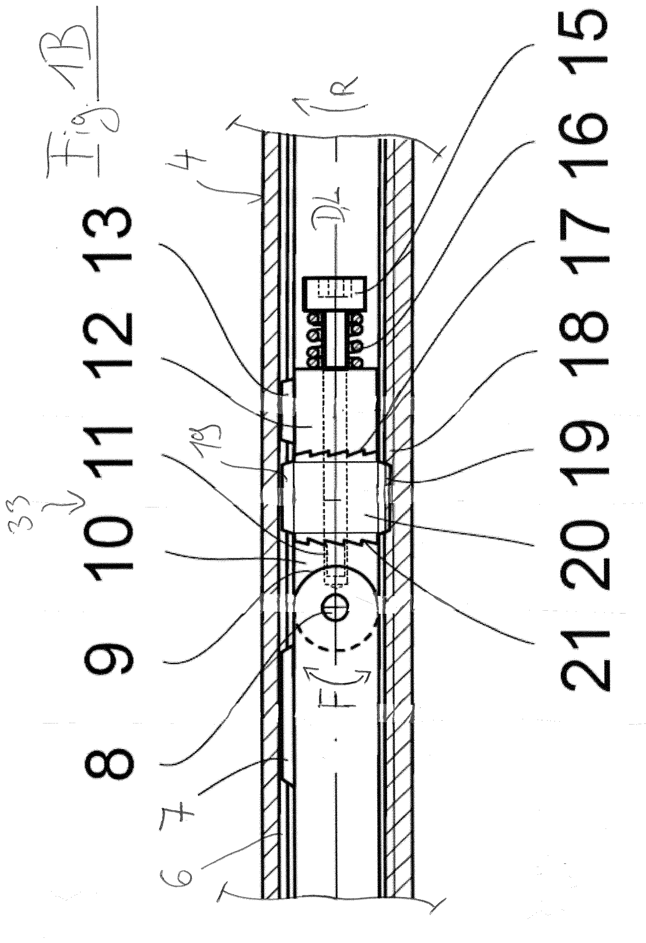

Die Stange 1 ist mittels einer Mutter 3 in Richtung des Befestigungsabschnitts 41 in dem Rohr 4 zentriert, wobei das Rohr ferner ein Innengewinde 18 und eine Nut 6 aufweist, in welcher eine an der Stange 1 ausgebildete Passfeder 7 eingreift. Die Passfeder 7 hat dabei in der Nut 6 ein geometrisches Spiel, so dass die Stange 1 im Rohr 4 um einen definierten Winkel α verdrehbar angeordnet ist, siehe Schnitt A-A. An einem dem Befestigungsabschnitt 41 gegenüberliegenden Ende der Stange 1 befindet sich ein mit einem als Bolzen ausgebildeten Kopplungselement 8 gesichertes Gelenk 9, das die Stange 1 an ein Antriebselement 10 koppelt, siehe Schnitt B-B und auch Fig. 1B. Dabei ist das Gelenk 9 von einem ersten, an der Stange 1 ausgebildeten Gelenkabschnitt 35 und einem zweiten, an dem Antriebselement 10 ausgebildeten Gelenkabschnitt 37 gebildet, wobei der konvexe erste Gelenkabschnitt 35 formschlüssig an dem zweiten konkaven Gelenkabschnitt 37 anliegt. Die Gelenkabschnitte 35 und 37 bilden damit ein zylindrisches Gelenk 9, welches einen rotatorischen Freiheitsgrad F zwischen der Stange 1 und dem Antriebselement 10 schafft.

Das Antriebselement 10 ist dabei eine Komponente einer Einstellmechanik 33 zur Anpassung der Länge des Marknagels 31 (vgl. hierzu auch Fig. 1B). An dem Antriebselement 10 greift ein Gewindeelement 20 mit einer Verzahnung 21 ein, die eine Freilaufrichtung und eine Sperrrichtung hat. Das Gewindeelement 20 weist ein Außengewinde 19 auf, das in das Innengewinde 18 des Rohres 4 eingeschraubt ist, siehe Schnitt C-C. Dadurch ist das Gewindeelement 20 drehbar um eine Drehachse D in dem Rohr 4 geführt, wobei eine Drehung des Gewindeelementes in Drehrichtung R in Abhängigkeit von der Gewindesteigung des Innengewindes 18 eine translatorische Bewegung des Gewindeelementes 20 in Richtung des Befestigungsabschnittes 41 oder von diesem weg zur Folge hat. Durch das Zusammenwirken bzw. den, zumindest mittelbaren, Kontakt zwischen dem Gewindeelement 20 und der Stange 1 kann diese translatorische Bewegung auf die Stange 1 übertragen werden, um eine Längenanpassung des Marknagels 31 zu erreichen.

Ferner greift das Antriebselement 20 mit einer Verzahnung 17 in ein Blockierelement 12 ein, das mit einer in der Nut 6 geführten Passfeder 13 axial verschiebbar in dem Rohr 4 angeordnet ist, siehe Schnitt D-D. Dabei ist die Ausdehnung der Passfeder 13 in Drehrichtung R größer als die der Passfeder 7 der Stange 1, so dass lediglich die Stange 1 um den Winkel α verdrehbar ist, während das Blockierelement 12 verdrehsicher in der Nut 6 geführt ist. Die Verzahnung 17 hat eine Freilaufrichtung und eine Sperrrichtung, die der Freilauf- und Sperrrichtung von Verzahnung 21 entgegen gerichtet ist. Das Blockierelement 12 und das Gewindeelement 20 sind mittels eines in das Antriebselement 10 an einer Verankerung 11 eingeschraubten Verbindungselementes, welches als Schraube 15 ausgebildet ist, und einer Feder 16 axial verspannt, so dass die Verzahnungen 21 und 17 sicher in Eingriff zueinander stehen.

Wird das Knochenteil, an dem die Stange 1 befestigt ist, um den sich aus dem Spiel der Passfeder 7 in der Nut 6 ergebenden Winkel α in Drehrichtung R verdreht, wird das Antriebselement 10 und über die in Sperrrichtung wirkende Verzahnung 21 auch das Gewindeelement 20 um den Winkel α verdreht. Aus der Steigung des Innengewindes 18 entsteht eine axiale Bewegung des Gewindeelementes 20 und in dessen Folge auch des Antriebselementes 10 und der damit verbundenen Stange 1. Das Blockierelement 12 wird durch die Passfederverbindung 13 am Mitdrehen gehindert, so dass die in Freilaufrichtung wirkende Verzahnung 17 einen Zahn überspringt. Die Feder 16 ermöglicht hierbei die axiale Bewegung und das sichere Einrasten des Blockierelements 12.

Wird die Stange 1 wieder entgegen der Drehrichtung R zurückgedreht, agiert die Verzahnung 21 in Freilaufrichtung und die Verzahnung 17 in Sperrrichtung, so dass das Gewindeelement 20 rotatorisch fixiert ist und die Verzahnung 21 einen Zahn überspringt. Der Marknagel 31 ist damit um ein sich aus der Gewindesteigung des Innengewindes 18 und dem Drehwinkel α der Stange 1 bestimmtes Inkrement axial verlängert oder verkürzt, je nach Gewindesteigung bzw. Drehrichtung R, und kann durch Wiederholen des Vorganges schrittweise weiter verlängert bzw. verkürzt werden.

Um eine der physiologischen Krümmung des Knochens entsprechende gekrümmte Ausführung des Marknagels 31 zu ermöglichen, sind die Stange 1 und die nach Druckschrift US5074882 in die Stange 1 direkt eingeschraubte Schraube 15 als Komponente der Einstellmechanik derart entkoppelt, dass eine Biegebeanspruchung der Schraube 15 durch die beim Vor- und Zurückdrehen der Stange 1 im Rohr 4 entstehende Winkelabweichung zwischen der Stange 1 und dem Gewindeelement 20 bzw. der Längsachse L der Schraube 15 verhindert wird, die ein hohes Risiko hinsichtlich eines Lösens der Schraubverbindung oder eines Bruchs der Schraube 15 darstellen würde. Bei stark gekrümmten Ausführungen des Marknagels 31 könnte zudem ein den Zahneingriff 21 negativ beeinflussender Winkelfehler zwischen der Stange 1 und dem Gewindeelement 20 entstehen.

Eine derartige Entkopplung von Stange 1 und Element 20 kann durch das in Fig.1A und 1B dargestellte Gelenk 9 realisiert werden, mit dem ein rotatorischer Freiheitsgrad F geschaffen wird, der die Entstehung eines Winkelfehlers und damit eines Biegemomentes auf die Schraube 15 beim Vor- und Zurückdrehen der Stange 1 verhindert. Das Kopplungselement 8 stellt vorzugsweise nur den Zusammenhalt des Gelenkes 9 sicher und ist durch ein entsprechend gewähltes Bolzenspiel in der Stange 1 oder in dem Antriebselement 10 nicht an der Übertragung der vom Marknagel 31 zu tragenden Axialkraft beteiligt. Die vom Marknagel 31 zu tragende Axialkraft wird vorzugsweise über die formschlüssige Anlage des ersten Gelenkabschnitts 35 an dem zweiten Gelenkabschnitt 37 an der Stange 1 und an dem Antriebselement 10 übertragen. Die Gelenkabschnitte 35 und 37 sind dabei vorzugsweise als Kreiskonturen zu gestalten. Das Kopplungselement 8 kann in bestimmten Anwendungen entfallen oder durch andere kraft- oder formschlüssige Lösungen ersetzt werden.

Bei der gekrümmten Ausführung des Marknagels 31 ist weiterhin ein radiales Spiel 5 zwischen der gekrümmten Stange 1 und dem gekrümmten Rohr 4 vorgehalten, um eine Beweglichkeit der Stange 1 im Rohr 4 beim Verdrehen der Stange 1 um die Drehachse D zu ermöglichen.

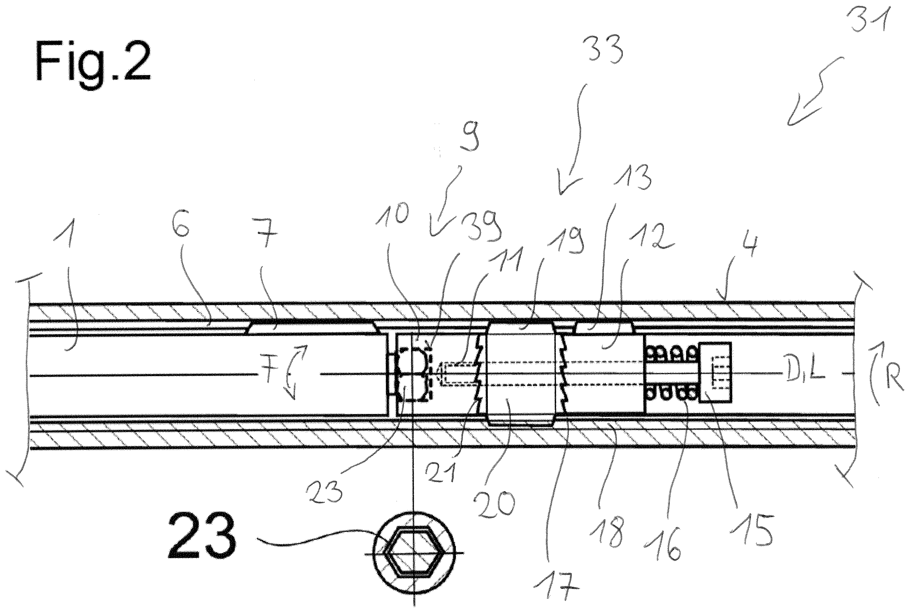

Fig. 2 zeigt eine weitere Ausführungsform eines Marknagels 31 bzw. dessen Einstellmechanik 33 sowie der Entkopplung zwischen der Stange 1 und dem Verbindungselement 15. Auch hierbei ist ein Gelenk 9 vorgesehen, welches die Stange 1 von der Einstellmechanik 33 und insbesondere dem als geradlinige Schraube ausgebildeten Verbindungselement 15 entkoppelt, um die Übertragung von Biegemomenten auf das Verbindungselement 15 zu verhindern. Wiederum ist das Verbindungselement 15 durch ein drehfest geführtes Blockierelement 12 und ein in einem Innengewinde 18 des Rohres 4 drehbar geführtes Gewindeelement 20 mit Außengewinde 19 hindurch laufend in einem Antriebselement 10 an einer Verankerung 11 verschraubt. Der Eingriff der Verzahnungen 17 und 21 ist durch ein zwischen dem Kopf der Schraube 15 und dem Blockierelement 12 wirkendes Federelement 16 sichergestellt.

Die Gelenkverbindung zwischen der Stange 1 und dem Antriebselement 10 erfolgt dabei mittels eines polygonalen Kopfes 23, welcher mit der Stange 1 verbunden ist und aus dieser in Richtung des Antriebselementes 10 herausragt. Insbesondere kann dies der Kopf 23 einer Sechskantschraube sein, welche in die Stange 1 eingeschraubt ist. Der polygonale Kopf 23 ist in einer Ausnehmung 39 des Antriebselements 10 mit geringfügigem Spiel gelagert, sodass die Stange 1 leicht gegenüber dem Antriebselement 10 auslenkbar ist und ein rotatorischer Freiheitsgrad F geschaffen wird, welcher die Übertragung von Biegemomenten auf das Verbindungselement 15 aufgrund der Krümmung des Marknagels 31 verhindert. Insbesondere kann die Ausnehmung 39 dabei korrespondierend zu einer Sechskantschraube als Innensechskant ausgebildet sein. Das Spiel des polygonalen Kopfes 23 in der Ausnehmung 39 kann dabei insbesondere so gewählt sein, dass eine Rotationsbewegung der Stange 1 um die Drehachse D in Drehrichtung R über den polygonalen Kopf 23 auf das Antriebselement 10 und über die Verzahnung 21 auf das Gewindeelement 20 übertragen werden kann. Ferner ist bei dieser Ausführungsform kein Kopplungselement 8 vorgesehen, wobei erkannt wurde, dass der axiale Zusammenhalt zwischen der Stange 1 und dem Rohr 4 bzw. der Eingriff des polygonalen Kopfes 23 in die Ausnehmung 39 bei eingesetztem Marknagel 31 durch die Muskelspannung zuverlässig gewährleistet werden kann.

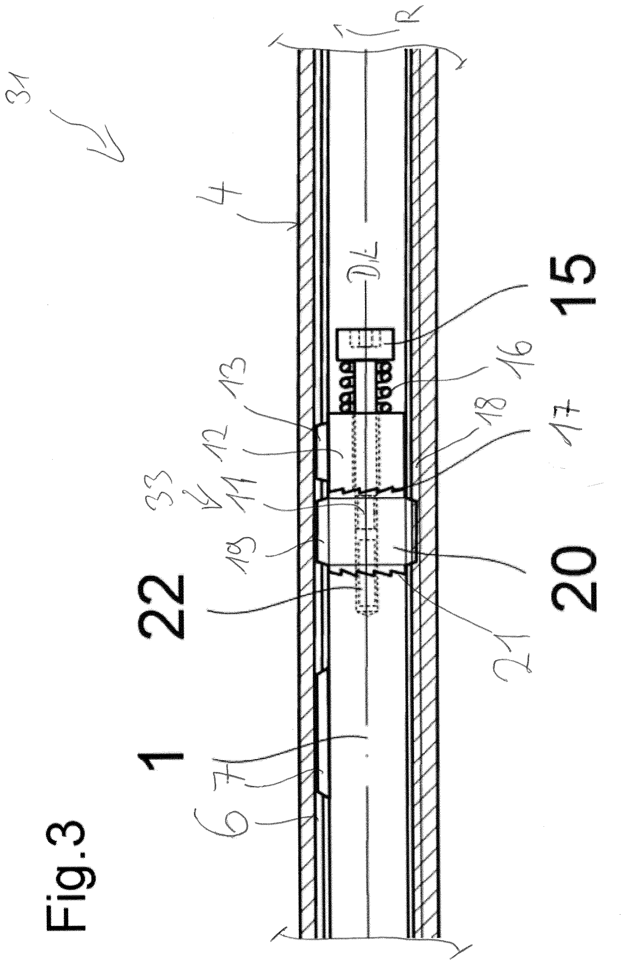

Fig. 3 zeigt eine weitere Möglichkeit der Entkopplung zwischen der Stange 1 und dem Verbindungselement 15, ohne dass ein Gelenk 9 vorgesehen ist. Darüber hinaus ist bei dieser Ausführungsform auch kein Antriebselement 10 vorgesehen, sondern die Stange 1 bildet die Verzahnung 21 direkt mit dem Gewindeelement 20.

Um dennoch eine Entkopplung zwischen der Stange 1 und dem Verbindungselement 15 bezüglich der Übertragung von Biegemomenten zu erreichen, ist das Verbindungselement 15, abweichend von bisher bekannten Lösungen, nicht in der Stange 1, sondern in dem Gewindeelement 20 an einer dafür vorgesehenen Verankerung 11 verschraubt. Eine Auslenkung der Stange 1 aufgrund der Krümmung des Marknagels 31 relativ zu der Drehachse D bzw. der Längsachse L des Verbindungselementes 15 kann folglich über das Gewindeelement 20 auf das Rohr 4 abgeleitet werden, ohne dass das Verbindungselement 15 belastet wird und der Gefahr eines Brechens ausgesetzt ist. Auch diese, zur Realisierung eines gekrümmten Marknagels 31 erforderliche Neuerung basiert insbesondere auf der Erkenntnis, dass eine Fixierung zwischen der Stange 1 und dem Gewindeelement 20 bzw. dem Blockierelement 12 nicht zwangsläufig erforderlich ist, sondern die Stabilität des Marknagels 31 auch durch die Gewebespannung zuverlässig gewährleistet werden kann.

Um den Eingriff der Verzahnung 21 trotz der Krümmung des Marknagels 31 in jeder Position zu gewährleisten und eine Auslenkung der Stange 1 gegenüber dieser Verzahnung 21 zu verhindern, ist ferner ein Zentrierstift 22 vorgesehen, welcher die Stange 1 axial bezüglich der Drehachse D des Gewindeelements 20 ausrichtet.

Erstmalig ermöglicht die hierin offenbarte Entkopplung zwischen der Stange 1 und Komponenten der Einstellmechanik 33, insbesondere dem Verbindungselement 15, somit den Einsatz eines gekrümmten Marknagels 31. Eine solche Krümmung, die insbesondere einer physiologischen Krümmung eines Röhrenknochens entsprechen kann, bietet weitreichende Vorteile, insbesondere beim Einbringen des Marknagels 31 in einen Markkanal während einer Operation.

### Bezugszeichenliste

- 1: Stange
- 2: Bohrung an der Stange
- 3: Mutter
- 4: Rohr
- 5: radiales Spiel
- 6: Nut
- 7: Passfeder der Stange
- 8: Kopplungselement
- 9: Gelenk
- 10: Antriebselement
- 11: Verankerung
- 12: Blockierelement
- 13: Passfeder des Blockierelementes
- 14: Bohrung an dem Rohr
- 15: Verbindungselement, Schraube
- 16: Spanneinrichtung, Feder
- 17: zweite Verzahnung
- 18: Innengewinde
- 19: Außengewinde
- 20: Gewindeelement
- 21: erste Verzahnung
- 22: Zentrierelement
- 23: polygonaler Kopf
- 31: gekrümmter Marknagel
- 33: Einstellmechanik
- 35: erster Gelenkabschnitt
- 37: zweiter Gelenkabschnitt
- 39: Ausnehmung
- 41: Befestigungsabschnitt
- D: Drehachse des Gewindeelementes
- F: rotatorischer Freiheitsgrad
- L: Längsachse des Verbindungselementes
- R: Drehrichtung

- α: vorgegebener Drehwinkel der Stange

## Patentansprüche

1. Gekrümmter Marknagel (31), insbesondere Distraktionsmarknagel oder Kontraktionsmarknagel, zum Verbinden zweier Teile eines Knochens, insbesondere zur Knochenverlängerung, zur Knochenverkürzung oder zur Überbrückung einer Knochenlücke, mit einer der physiologischen Krümmung des Knochens entsprechenden Krümmung,
mit einem Rohr (4) zum Befestigen an dem ersten Knochenteil,
mit einer innerhalb des Rohres (4) angeordneten Stange (1), welche einen aus dem Rohr (4) herausragenden Befestigungsabschnitt (41) zum Befestigen an dem zweiten Knochenteil aufweist, und
mit einer innerhalb des Rohres (4) angeordneten Einstellmechanik (33), welche durch wiederholte relative Rotationsbewegungen zwischen der Stange (1) und dem Rohr (4) betätigbar ist,
wobei die Stange (1) durch Betätigen der Einstellmechanik (33) aus dem Rohr (4) herausfahrbar oder in das Rohr (4) hineinfahrbar ist, und
wobei eine Entkopplung zwischen der Stange (1) und zumindest einer Komponente der Einstellmechanik (33) vorgesehen ist, welche die Übertragung von krümmungsbedingten Biegemomenten auf die Komponente verhindert.

2. Gekrümmter Marknagel (31) nach Anspruch 1,
wobei das Rohr (4) ein Innengewinde (18) aufweist und die Einstellmechanik (33) ein in dem Innengewinde (18) um eine Drehachse (D) drehbares Gewindeelement (20) umfasst.

3. Gekrümmter Marknagel (31) nach Anspruch 2,
wobei das Gewindeelement (20) über eine erste Verzahnung (21) mit der Stange (1) in Eingriff steht,
insbesondere wobei eine Rotationsbewegung der Stange (1) um die Drehachse (D) entlang einer Drehrichtung (R) über die erste Verzahnung (21) auf das Gewindeelement (20) übertragbar ist,
und/oder wobei die Stange (1) eine Passfeder (7) aufweist, welche in einer Nut (6) des Rohres (4) geführt ist, oder umgekehrt, wobei die Nut (6) eine Rotationsbewegung der Stange (1) um die Drehachse (D) begrenzt.

4. Gekrümmter Marknagel (31) nach Anspruch 2 oder 3,
wobei das Gewindeelement (20) an einer der Stange (1) abgewandten Seite über eine zweite Verzahnung (17) mit einem Blockierelement (12) in Eingriff steht, wobei die zweite Verzahnung (17) entgegen der Drehrichtung (R) eine Sperrrichtung aufweist, welche eine Rotation des Gewindeelementes (20) relativ zu dem Blockierelement (12) entgegen der Drehrichtung (R) blockiert, und wobei die zweite Verzahnung (17) in Drehrichtung (R) eine Freilaufrichtung aufweist, welche eine Rotation des Gewindeelementes (20) relativ zu dem Blockierelement (12) in Drehrichtung (R) ermöglicht, insbesondere wobei die erste Verzahnung (21) entgegen der Drehrichtung (R) eine Freilaufrichtung aufweist, welche ein Zurückdrehen der Stange (1) entgegen der Drehrichtung (R) bei durch das Blockierelement (12) blockiertem Gewindeelement (20) ermöglicht.

5. Gekrümmter Marknagel (31) nach Anspruch 4,
wobei das Blockierelement (12) verdrehsicher bezüglich der Drehachse (D) innerhalb des Rohres (4) geführt ist, insbesondere wobei das Blockierelement (12) eine Passfeder (13) aufweist, welche innerhalb des Rohres (4) in einer Nut (6) geführt ist, oder umgekehrt,
und/oder wobei das Blockierelement (12) in Eingriff mit dem Gewindeelement (20) vorgespannt und gegen die Rückstellkraft der Vorspannung au-ßer Eingriff mit dem Gewindeelement (20) bringbar ist.

6. Gekrümmter Marknagel (31) nach Anspruch 4 oder 5,
wobei das Blockierelement (12) mittels eines geradlinigen Verbindungselements (15), insbesondere einer Schraube, welches sich durch das Blockierelement (12) hindurch erstreckt, in Eingriff mit dem Gewindeelement (20) vorgespannt ist,
insbesondere wobei zwischen dem Verbindungselement (15) und dem Blockierelement (12) eine Spanneinrichtung (16), insbesondere einer Feder, wirksam ist, welche die Vorspannung auf das Blockierelement (12) ausübt.

7. Gekrümmter Marknagel (31) nach Anspruch 6,
wobei sich das Verbindungselement (15) parallel zu der Drehachse (D) erstreckt, wobei insbesondere eine Längsachse (L) des Verbindungselementes (15) mit der Drehachse (D) zusammenfällt,
und/oder wobei das Verbindungselement (15) als Komponente der Einstellmechanik (33) in dem Gewindeelement (20) verankert ist und hierdurch die Entkopplung zwischen Stange (1) und Verbindungselement (15) erfolgt, und/oder wobei das Gewindeelement (20) über ein insbesondere stiftförmiges Zentrierelement (22) mit der Stange (1) verbunden ist, welches die Stange (1) axial bezüglich der Drehachse (D) zu dem Gewindeelement (20) ausrichtet.

8. Gekrümmter Marknagel (31) nach einem der Ansprüche 5 bis 7, wobei zur Entkopplung der Stange (1) von dem eine Komponente der Einstellmechanik (33) bildenden Verbindungselement (15) ein Gelenk (9) vorgesehen ist, welches zumindest einen rotatorischen Freiheitsgrad (F) zwischen dem Verbindungselement (15) und der Stange (1) schafft, insbesondere wobei das Gelenk (9) zumindest einen rotatorischen Freiheitsgrad (F) bezüglich Drehbewegungen um zumindest eine senkrecht zu der Drehachse (D) verlaufende Achse schafft.

9. Gekrümmter Marknagel (31) nach Anspruch 8,
wobei das Gelenk (9) die Stange (1) mit einem Antriebselement (10) drehfest bezüglich Rotationsbewegungen um die Drehachse (D) verbindet, insbesondere wobei das Antriebselement (10) die erste Verzahnung (21) mit dem Gewindeelement (20) bildet.

10. Gekrümmter Marknagel (31) nach Anspruch 9,
wobei das Antriebselement (10) eine Passfeder (7) aufweist, welche in einer Nut (6) des Rohres (4) geführt ist, oder umgekehrt, wobei die Nut (6) die Rotationsbewegung des Antriebselements (10) um die Drehachse begrenzt (D),
und/oder wobei das Verbindungselement (15) sich durch das Blockierelement (12) hindurch erstreckt und in dem Antriebselement (10) verankert ist.

11. Gekrümmter Marknagel (31) nach Anspruch 9 oder 10,
wobei das Gelenk (9) einen ersten an der Stange (1) ausgebildeten Gelenkabschnitt (35) und einen zweiten an dem Antriebselement (10) ausgebildeten Gelenkabschnitt (37) umfasst, wobei der erste Gelenkabschnitt (35) formschlüssig mit dem zweiten Gelenkabschnitt (37) verbunden ist, insbesondere wobei der erste Gelenkabschnitt (35) konvex und der zweite Gelenkabschnitt (37) konkav - oder umgekehrt - ausgebildet ist, insbesondere wobei der erste Gelenkabschnitt (35) und der zweite Gelenkabschnitt (37) im Querschnitt kreisförmig ausgebildet sind.

12. Gekrümmter Marknagel (31) nach einem der Ansprüche 9 bis 11,
wobei die Stange (1) über ein Kopplungselement (8), insbesondere einen Bolzen, mit dem Antriebselement (10) verbunden ist,
insbesondere wobei das Kopplungselement (8) mit Spiel in der Stange (1) und/oder dem Antriebselement (10) gelagert ist.

13. Gekrümmter Marknagel (31) nach einem der Ansprüche 9 bis 12,
wobei das Gelenk (9) einen polygonalen Kopf (23) umfasst, welcher mit der Stange (1) verbunden ist und in einer an dem Antriebselement (10) ausgebildeten Ausnehmung (39) gegen die Drehachse (D) verschwenkbar gelagert ist.

14. Gekrümmter Marknagel (31) nach einem der Ansprüche 8 bis 13,
wobei das Gelenk (9) als Kardangelenk ausgebildet ist.

15. Gekrümmter Marknagel (31) nach einem der vorhergehenden Ansprüche, wobei die Stange (1) mit einem radialen Spiel (5) innerhalb des Rohres (4) angeordnet ist,
und/oder wobei die Rotationsbewegungen zum Betätigen der Einstellmechanik (33) manuell erzeugbar sind, insbesondere dann, wenn das Rohr (4) und die Stange (1) an den jeweiligen Knochenteilen befestigt sind.

## Claims

1. A curved intramedullary nail (31), in particular a distraction intramedullary nail or a contraction intramedullary nail, for connecting two parts of a bone, in particular for bone extension, for bone shortening or for bridging a bone gap, said curved intramedullary nail (31) comprising a curvature corresponding to the physiological curvature of the bone;
a tube (4) for fastening to the first bone part;
a rod (1) which is arranged within the tube (4) and which has a fastening section (41) projecting from the tube (4) for fastening to the second bone part; and
a setting mechanism (33) which is arranged within the tube (4) and which can be actuated by repeated relative rotational movements between the rod (1) and the tube (4),
wherein the rod (1) can be moved out of the tube (4) or can be moved into the tube (4) by actuating the setting mechanism (33), and
wherein a decoupling is provided between the rod (1) and at least one component of the setting mechanism (33) and prevents the transmission of curvature-induced bending moments to the component.

2. A curved intramedullary nail (31) in accordance with claim 1,
wherein the tube (4) has an internal thread (18) and the setting mechanism (33) comprises a threaded element (20) which is rotatable in the internal thread (18) about an axis of rotation (D).

3. A curved intramedullary nail (31) in accordance with claim 2,
wherein the threaded element (20) is in engagement with the rod (1) via a first toothed arrangement (21),
in particular wherein a rotational movement of the rod (1) about the axis of rotation (D) along a direction of rotation (R) can be transmitted to the threaded element (20) via the first toothed arrangement (21), and/or wherein the rod (1) has a key (7) which is guided in a groove (6) of the tube (4), or vice versa, wherein the groove (6) limits a rotational movement of the rod (1) about the axis of rotation (D).

4. A curved intramedullary nail (31) in accordance with claim 2 or claim 3, wherein the threaded element (20) is in engagement with a blocking element (12) via a second toothed arrangement (17) at a side facing away from the rod (1), wherein the second toothed arrangement (17) has a blocking direction against the direction of rotation (R), said blocking direction blocking a rotation of the threaded element (20) relative to the blocking element (12) against the direction of rotation (R), and wherein the second toothed arrangement (17) has a freewheeling direction in the direction of rotation (R), said freewheeling direction enabling a rotation of the threaded element (20) relative to the blocking element (12) in the direction of rotation (R),
in particular wherein the first toothed arrangement (21) has a freewheeling direction against the direction of rotation (R), said freewheeling direction enabling a rotating back of the rod (1) against the direction of rotation (R) when the threaded element (20) is blocked by the blocking element (12).

5. A curved intramedullary nail (31) in accordance with claim 4,
wherein the blocking element (12) is guided in a manner secure against rotation with respect to the axis of rotation (D) within the tube (4), in particular wherein the blocking element (12) has a key (13) which is guided within the tube (4) in a groove (6), or vice versa,
and/or wherein the blocking element (12) is preloaded in engagement with the threaded element (20) and can be brought out of engagement with the threaded element (20) against the return force of the preload.

6. A curved intramedullary nail (31) in accordance with claim 4 or claim 5,
wherein the blocking element (12) is preloaded in engagement with the threaded element (20) by means of a rectilinear connection element (15), in particular a screw, which extends through the blocking element (12),
in particular wherein a tensioning device (16), in particular a spring, is effective between the connection element (15) and the blocking element (12) and exerts the preload on the blocking element (12).

7. A curved intramedullary nail (31) in accordance with claim 6,
wherein the connection element (15) extends in parallel with the axis of rotation (D), wherein in particular a longitudinal axis (L) of the connection element (15) coincides with the axis of rotation (D),
and/or wherein the connection element (15) is anchored in the threaded element (20) as a component of the setting mechanism (33) and the decoupling between the rod (1) and the connection element (15) hereby takes place,
and/or wherein the threaded element (20) is connected to the rod (1) via a centering element (22), in particular a pin-shaped centering element (22), which axially aligns the rod (1) with the threaded element (20) with respect to the axis of rotation (D).

8. A curved intramedullary nail (31) in accordance with any one of the claims 5 to 7,
wherein, for decoupling the rod (1) from the connection element (15) forming a component of the setting mechanism (33), a joint (9) is provided which provides at least one rotational degree of freedom (F) between the connection element (15) and the rod (1),
in particular wherein the joint (9) provides at least one rotational degree of freedom (F) with respect to rotational movements about at least one axis extending perpendicular to the axis of rotation (D).

9. A curved intramedullary nail (31) in accordance with claim 8,
wherein the joint (9) connects the rod (1) to a drive element (10) in a rotationally fixed manner with respect to rotational movements about the axis of rotation (D),
in particular wherein the drive element (10) forms the first toothed arrangement (21) with the threaded element (20).

10. A curved intramedullary nail (31) in accordance with claim 9,
wherein the drive element (10) has a key (7) which is guided in a groove (6) of the tube (4), or vice versa, wherein the groove (6) limits the rotational movement of the drive element (10) about the axis of rotation (D),
and/or wherein the connection element (15) extends through the blocking element (12) and is anchored in the drive element (10).

11. A curved intramedullary nail (31) in accordance with claim 9 or claim 10,
wherein the joint (9) comprises a first joint section (35) formed at the rod (1) and a second joint section (37) formed at the drive element (10), wherein the first joint section (35) is connected in a form-fitting manner to the second joint section (37),
in particular wherein the first joint section (35) is convex and the second joint section (37) is concave - or vice versa - in particular wherein the first joint section (35) and the second joint section (37) are circular in cross-section.

12. A curved intramedullary nail (31) in accordance with any one of the claims 9 to 11,
wherein the rod (1) is connected to the drive element (10) via a coupling element (8), in particular a pin,
in particular wherein the coupling element (8) is supported with clearance in the rod (1) and/or the drive element (10).

13. A curved intramedullary nail (31) in accordance with any one of the claims 9 to 12,
wherein the joint (9) comprises a polygonal head (23) which is connected to the rod (1) and which is pivotably supported against the axis of rotation (D) in a recess (39) formed at the drive element (10).

14. A curved intramedullary nail (31) in accordance with any one of the claims 8 to 13,
wherein the joint (9) is configured as a gimbal joint.

15. A curved intramedullary nail (31) in accordance with any one of the preceding claims,
wherein the rod (1) is arranged with a radial clearance (5) within the tube (4),
and/or wherein the rotational movements for actuating the setting mechanism (33) can be manually generated, in particular when the tube (4) and the rod (1) are fastened to the respective bone parts.

## Revendications

1. Clou intramédullaire incurvé (31), en particulier clou intramédullaire de distraction ou clou intramédullaire de contraction, destiné à relier deux parties d'un os, en particulier à allonger un os, à raccourcir un os ou à combler une lacune inter-osseuse, présentant une courbure qui correspond à la courbure physiologique de l'os,
comprenant
un tube (4) destiné à être fixé à la première partie de l'os,
une tige (1) disposée à l'intérieur du tube (4), qui présente une portion de fixation (41) dépassant du tube (4) et destinée à être fixée à la deuxième partie de l'os, et
un mécanisme de réglage (33) disposé à l'intérieur du tube (4), qui peut être actionné par des mouvements de rotation relatifs répétés entre la tige (1) et le tube (4),
dans lequel
la tige (1) peut être extraite du tube (4) ou introduite dans le tube (4) par actionnement du mécanisme de réglage (33), et
il est prévu un découplage entre la tige (1) et au moins un composant du mécanisme de réglage (33), qui empêche la transmission de couples de flexion, dus à la courbure, au composant.

2. Clou intramédullaire incurvé (31) selon la revendication 1,
dans lequel le tube (4) présente un taraudage (18) et le mécanisme de réglage (33) comprend un élément fileté (20) pouvant tourner autour d'un axe de rotation (D) dans le taraudage (18).

3. Clou intramédullaire incurvé (31) selon la revendication 2,
dans lequel l'élément fileté (20) est en prise avec la tige (1) par l'intermédiaire d'une première denture (21),
en particulier, un mouvement de rotation de la tige (1) autour de l'axe de rotation (D) selon un sens de rotation (R) peut être transmis à l'élément fileté (20) par l'intermédiaire de la première denture (21),
et/ou la tige (1) comporte une clavette (7) qui est guidée dans une rainure (6) du tube (4), ou inversement, la rainure (6) limitant un mouvement de rotation de la tige (1) autour de l'axe de rotation (D).

4. Clou intramédullaire incurvé (31) selon la revendication 2 ou 3,
dans lequel, sur un côté détourné de la tige (1), l'élément fileté (20) est en prise avec un élément de blocage (12) par l'intermédiaire d'une deuxième denture (17), la deuxième denture (17) présentant, en sens opposé au sens de rotation (R), un sens de blocage qui bloque une rotation de l'élément fileté (20) par rapport à l'élément de blocage (12) en sens opposé au sens de rotation (R), et la deuxième denture (17) présentant, dans le sens de rotation (R), un sens en roue libre qui permet une rotation de l'élément fileté (20) par rapport à l'élément de blocage (12) dans le sens de rotation (R), et en particulier, la première denture (21) présente, en sens opposé au sens de rotation (R), un sens en roue libre qui permet un retour de la tige (1) en sens opposé au sens de rotation (R) lorsque l'élément fileté (20) est bloqué par l'élément de blocage (12).

5. Clou intramédullaire incurvé (31) selon la revendication 4,
dans lequel l'élément de blocage (12) est guidé à l'intérieur du tube (4) sans pouvoir tourner par rapport à l'axe de rotation (D), en particulier l'élément de blocage (12) présente une clavette (13) qui est guidée à l'intérieur du tube (4) dans une rainure (6), ou inversement,
et/ou l'élément de blocage (12) est précontraint en prise avec l'élément fileté (20) et peut être dégagé de l'élément fileté (20) à l'encontre de la force de rappel de la précontrainte.

6. Clou intramédullaire incurvé (31) selon la revendication 4 ou 5,
dans lequel l'élément de blocage (12) est précontraint en prise avec l'élément fileté (20) au moyen d'un élément de liaison rectiligne (15), en particulier au moyen d'une vis, qui s'étend à travers l'élément de blocage (12), en particulier, un organe de contrainte (16), en particulier un ressort, agit entre l'élément de liaison (15) et l'élément de blocage (12), lequel exerce la précontrainte sur l'élément de blocage (12).

7. Clou intramédullaire incurvé (31) selon la revendication 6,
dans lequel l'élément de liaison (15) s'étend parallèlement à l'axe de rotation (D), en particulier, un axe longitudinal (L) de l'élément de liaison (15) coïncide avec l'axe de rotation (D),
et/ou l'élément de liaison (15) en tant que composant du mécanisme de réglage (33) est ancré dans l'élément fileté (20), ce qui permet le découplage entre la tige (1) et l'élément de liaison (15),
et/ou l'élément fileté (20) est relié à la tige (1) par un élément de centrage (22), en particulier en forme de broche, qui oriente la tige (1) par rapport à l'élément fileté (20) axialement par rapport à l'axe de rotation (D).

8. Clou intramédullaire incurvé (31) selon l'une des revendications 5 à 7, dans lequel, pour découpler la tige (1) de l'élément de liaison (15) constituant un composant du mécanisme de réglage (33), il est prévu une articulation (9) qui crée au moins un degré de liberté en rotation (F) entre l'élément de liaison (15) et la tige (1),
en particulier, l'articulation (9) fournit au moins un degré de liberté en rotation (F) par rapport aux mouvements de rotation autour d'au moins un axe perpendiculaire à l'axe de rotation (D).

9. Clou intramédullaire incurvé (31) selon la revendication 8,
dans lequel l'articulation (9) relie la tige (1) à un élément d'entraînement (10) solidairement en rotation par rapport à des mouvements de rotation autour de l'axe de rotation (D),
en particulier, l'élément d'entraînement (10) constitue la première denture (21) avec l'élément fileté (20).

10. Clou intramédullaire incurvé (31) selon la revendication 9,
dans lequel l'élément d'entraînement (10) présente une clavette (7) qui est guidée dans une rainure (6) du tube (4), ou inversement, la rainure (6) limitant le mouvement de rotation de l'élément d'entraînement (10) autour de l'axe de rotation (D),
et/ou l'élément de liaison (15) s'étend à travers l'élément de blocage (12) et est ancré dans l'élément d'entraînement (10).

11. Clou intramédullaire incurvé (31) selon la revendication 9 ou 10, dans lequel l'articulation (9) comprend une première portion d'articulation (35) formée sur la tige (1) et une deuxième portion d'articulation (37) formée sur l'élément d'entraînement (10), la première portion d'articulation (35) étant reliée par coopération de forme à la deuxième portion d'articulation (37),
en particulier, la première portion d'articulation (35) est convexe et la deuxième portion d'articulation (37) est concave - ou inversement -,
en particulier, la première portion d'articulation (35) et la deuxième portion d'articulation (37) ont une section transversale circulaire.

12. Clou intramédullaire incurvé (31) selon l'une des revendications 9 à 11,
dans lequel la tige (1) est reliée à l'élément d'entraînement (10) par un élément de couplage (8), en particulier par un goujon,
en particulier, l'élément de couplage (8) est logé avec jeu dans la tige (1) et/ou dans l'élément d'entraînement (10).

13. Clou intramédullaire incurvé (31) selon l'une des revendications 9 à 12, dans lequel l'articulation (9) comprend une tête polygonale (23) qui est reliée à la tige (1) et qui est logée de façon mobile en pivotement par rapport à l'axe de rotation (D) dans un évidement (39) ménagé sur l'élément d'entraînement (10).

14. Clou intramédullaire incurvé (31) selon l'une des revendications 8 à 13, dans lequel l'articulation (9) est réalisée sous forme de joint de cardan.

15. Clou intramédullaire incurvé (31) selon l'une des revendications précédentes,
dans lequel la tige (1) est disposée avec un jeu radial (5) à l'intérieur du tube (4),
et/ou les mouvements de rotation pour actionner le mécanisme de réglage (33) peuvent être générés manuellement, en particulier lorsque le tube (4) et la tige (1) sont fixés aux parties respectives de l'os.
